# EUROPEAN PATENT SPECIFICATION

(11) **EP 4 665 233 B1**
(45) Date of publication and mention of the grant of the patent: **02.12.2026**
(21) Application number: 24702426.8
(22) Date of filing: 23.01.2024
(51) Int. Cl.: A61B 5/363, A61B 5/367, A61B 5/00

(54) **VARIATION IN ELECTROPHYSIOLOGICAL SIGNALS ACROSS SURFACES AND RELATED MAPS**
VARIATION VON ELEKTROPHYSIOLOGISCHEN SIGNALEN ÜBER OBERFLÄCHEN HINWEG UND ZUGEHÖRIGE KARTEN
VARIATION DE SIGNAUX ÉLECTROPHYSIOLOGIQUES ENTRE DES SURFACES ET CARTES ASSOCIÉES

(30) Priority: 13.02.2023 US 202363484652 P
(43) Date of publication of application: 24.12.2025
(73) Proprietor: CardioInsight Technologies, Inc., Independence, OH 44131 (US)
(72) Inventor: ZENG, Qingguo, Minneapolis, Minnesota 55432 (US); LOU, Qing, Minneapolis, Minnesota 55432 (US); LASKE, Timothy G., Minneapolis, Minnesota 55432 (US); DINICOLA, Michelle K., Minneapolis, Minnesota 55432 (US)
(74) Representative: Zimmermann & Partner Patentanwälte mbB
(86) International application number: PCT/IB2024/050631
(87) International publication number: WO 2024/170970

(56) References cited:
- US-A1- 2012 089 038
- US-B1- 6 236 883
- KENICHI HARUMI: "The Processed Map of the Body Surface Potential", JAPANESE CIRCULATION JOURNAL, vol. 45, no. 10, 20 October 1981 (1981-10-20), pages 1179 - 1181, XP093141321, ISSN: 0047-1828, DOI: https://doi.org/10.1253/jcj.45.1179

## Description

### CROSS-REFERENCE TO RELATED APPLICATION

This application claims priority to U.S. Provisional Patent Application No. 63/484,652, filed February 13, 2023.

### FIELD

The present technology is generally related to determining variation in electrophysiological signals across surfaces and generating related maps.

### BACKGROUND

Ventricular tachycardia (VT) is a fast heart rate (e.g., more than 100 beats per minute) that starts in the heart's lower chambers (ventricles). Electrocardiograms (ECGs) can be used in VT mapping, such as mapping to help identify a chamber of origin of VT and/or exit sites of VT. However, such VT mapping has had minimal clinical or diagnostic value in mapping of VT circuits, including scar-related VT circuits US6236883 B1 and KENICHI HARUMI: "The Processed Map of the Body Surface Potential", JAPANESE CIRCULATION JOURNAL, vol. 45, no. 10, 20 October 1981 (1981-10-20), pages 1179-1181, ISSN: 0047-1828, DOI: https://doi.org/10.1253/jcj.45.1179 relate to cardiac variance maps.

### SUMMARY

The techniques of this disclosure generally relate to determining variation in electrophysiological signals across surfaces and generating related maps.

In one aspect, the present disclosure provides a method. The method includes selecting multiple sets of beat intervals, in which each set of beat intervals includes respective cardiac electrophysiological signals at respective locations across a surface of interest for the respective beat interval. The method also includes determining an average beat interval for each set of beat intervals based on the respective cardiac electrophysiological signals in the respective set of beat intervals. The method also includes determining, for each set of beat intervals, a difference between each of the respective cardiac electrophysiological signals and the average beat interval for each respective set of beat intervals. The method also includes computing a measure of variation for each of the respective locations across the surface of interest based on the average beat interval and the differences determined for the respective locations across the surface of interest. The method also includes generating a variation map across the surface of interest based on the measure of variation computed for each of the respective locations.

In another aspect, the disclosure provides one or more non-transitory computer-readable media having instructions, which when executed by a processor, perform a method described herein.

In another aspect, the disclosure provides a system. The system can include one or more non-transitory computer-readable media to store data and machine-readable instructions, in which the data includes electrophysiological data representative of cardiac electrophysiological signals at respective locations across a surface of interest. A processing unit can access the non-transitory computer-readable media and execute the machine-readable instructions. The machine-readable instructions can include interval selector code, average calculator code, difference calculator code, and variation calculator code. The interval selector code is programmed to select multiple sets of beat intervals, in which each set of beat intervals includes respective cardiac electrophysiological signals at respective locations across the surface of interest for the respective beat interval. The average calculator code is programmed to determine an average beat interval for each set of beat intervals based on the respective cardiac electrophysiological signals in the respective set of beat intervals. The difference calculator code is programmed to determine, for each set of beat intervals, a difference between each of the respective cardiac electrophysiological signals and the average beat interval for each respective set of beat intervals. The variation calculator code is programmed to compute a measure of variation for each of the respective locations across the surface of interest based on the average beat interval and the differences determined for the respective beat intervals across the surface of interest, in which a variation map across the surface of interest is generated based on the measure of variation computed for each of the respective locations.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a block diagram showing an example of a system 100 for monitoring and mapping cardiac EP information.
FIG. 2 is a block diagram that illustrates another example system having a mapping system and a therapy system.
FIG. 3 is a flow diagram showing an example method of generating variation maps.
FIG. 4 is a flow diagram showing an example method of treating a patient.
FIG. 5 is a signal diagram showing example EP signals.
FIG. 6 is a signal diagram showing an example of an EP signal at a given location.
FIG. 7 is a signal diagram showing another example of an EP signal at a given location with another physiological signal for gating purposes.
FIG. 8 is a signal diagram showing signal waveforms in multiple sets of beat intervals.
FIG. 9 is a signal diagram showing difference signal waveforms for respective sets of beat intervals.
FIGS. 10A, 10B and 10C depict examples of graphical variation maps.
FIGS. 11A, 11B and 11C depict examples of different types of variation maps.

### DETAILED DESCRIPTION

This disclosure relates to determining variation in electrophysiological (EP) signals across surfaces and generating related maps.

In some examples, the variation in EP signals (e.g., beat-to-beat variations) can be used to generate one or more variation maps. The variation maps can identify variations in EP activity across a surface of interest, including on an outer surface of a patient's body, on a cardiac surface of interest (e.g., epicardial, endocardial) or a combination of surfaces. The variation maps thus can represent ventricular tachycardia (VT) circuits, including through scar regions, which can alter the path of VT EP signals. The systems and methods described herein advantageously can provide additional insight not available with many existing ECG systems.

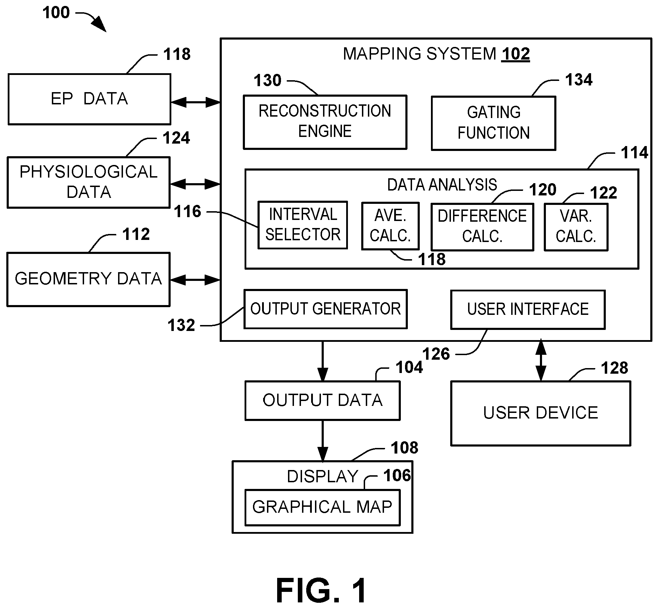

FIG. 1 depicts an example of a system 100 for monitoring and mapping cardiac EP information measured from a patient's body over time. The system 100 includes a mapping system 102 configured to generate output data 104, which may be used to render one or more graphical maps (e.g., a map on anatomical model) 106 and/or display processed electrical signals on a display 108. The mapping system 102 can be a computer-implemented apparatus that includes one or more processors and memory (one or more non-transitory computer-readable media) to store data and machine-readable instructions, which are executable by the processor to perform respective functions described herein. In another example, the mapping system 102 can be machine-readable instructions, which are stored in one or more non-transitory media and are executable by the processor to perform respective functions described herein. The mapping system 102 can also provide information in other display formats to provide guidance to a user (e.g., person and/or machine) representative of and/or derived from EP data 110.

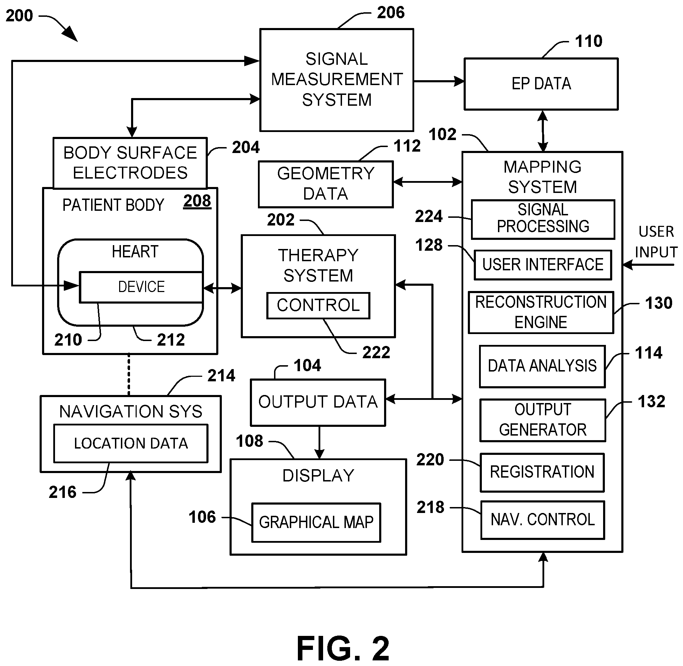

The EP data 110 includes information representative of electrophysiological signals across one or more surfaces of interest acquired over one or more time intervals. The EP data 110 can be measured directly from a patient's body, such as by an arrangement of electrodes distributed across an outer surface patient's torso (e.g., thorax), and/or measured invasively by electrodes (e.g., contact or non-contact electrodes) on or within a patient's heart, such as described herein (see, e.g., FIG. 2). Alternatively or additionally, the EP data 110 can represent signals across a surface of interest (e.g., a cardiac surface), which has been derived from EP signal measurements across a different surface (e.g., by performing inverse reconstruction). The EP data 110 can include unipolar, bipolar or a combination of unipolar and bipolar electrophysiological signals depending on the configuration of electrodes and processing of the measured signals. For example, the EP data includes pre-processed signals, which pre-processing can include removal of bad signal channels and/or filtering (e.g., low pass, high pass, and/or band pass filtering). The EP data 110 thus can include signal measurement values for each sample as well as additional information, such as timestamps and channel information.

The mapping system 102 is programmed to generate the output data 104 based on the EP data 110 and geometry data 112. The geometry data 112 can represent spatial locations in three-dimensional space for each of the EP signals, which are represented in the EP data 110, across each surface(s) of interest where the EP signals are measured or derived. As described herein, the geometry data thus can represent spatial locations (e.g. locations of non-invasive electrodes) distributed across the outer surface of a patient's body and/or locations distributed across one or more surfaces within the patient's body. The geometry data 112 can include electrode geometry data and anatomical geometry data. While the EP data 110 and geometry data 112 are shown as separate blocks, such data can be stored in memory in a common data structure integrating spatial and electrical signals across each surface of interest.

As an example, the geometry data 112 can be derived from imaging data acquired by a three-dimensional medical imaging modality. In one example, an anatomical model can be constructed based on imaging data obtained (e.g., by a medical imaging modality) for the patient to provide spatial coordinates for points across the patient's heart and, in some cases, in which the electrodes are positioned on the patient's body when the medical image is acquired, for the locations of the body surface electrodes positioned on the outer surface of the patient's body. The medical imaging data can be generated for the patient's body using a medical imaging modality, such as multi-plane x-ray, computed tomography (CT), magnetic resonance imaging (MRI), ultrasound, positron emission tomography (PET), single-photon emission computed tomography (SPECT) and the like. The electrode locations and locations of the surface (or surfaces) of interest can be identified in a respective coordinate system of the acquired images through appropriate image processing, including extraction and segmentation. For instance, segmented image data can be converted into a two-dimensional or three-dimensional graphical representation that includes the volume of interest for the patient. Appropriate anatomical or other landmarks can be identified in the geometry data 112 to facilitate spatial registration of the EP data 110. The identification of such landmarks can be done manually (e.g., by a person via image editing software) and/or automatically (e.g., via image processing techniques). In another example, the location of the body surface electrodes can be acquired by a digitizer, manual measurements or another non-imaging based technique (e.g., a navigation system - see, e.g., FIG. 2). The anatomical geometry may be implemented as a mathematical model (e.g., a spline or mesh or point cloud) that defines locations (e.g., a point cloud) across the surface of interest. The electrode geometry can also be implemented as a model that defines spatial coordinates of the electrodes in a common coordinate system.

The mapping system 102 includes data analysis code 114 programmed to analyze the EP data 110 and generate respective maps, such as described herein. The data analysis code 114 can include instructions executable by one or more processors to perform various functions disclosed herein. In the example of FIG. 1, the data analysis code 114 includes interval selector code 116, average calculator code 118, difference calculator code 120 and variation calculator code 122. Thus, the data analysis code 114 can perform calculations and associated functions based on EP data 110, the geometry data 112 as well as based on other physiological data 124, such as described herein.

The interval selector 116 is programmed to select multiple sets of beat intervals based on the EP signals at respective locations distributed across the surface (or surfaces) of interest. The interval selector 116 can identify the respective beat intervals from the signals represented by the EP data 110, which can include body surface EP signal measurements, reconstructed electrophysiological signals (e.g., provided by reconstruction engine 130 - see below) or from a combination of body surface and reconstructed EP signals. The body surface signals can be measured using the same electrodes or a subset thereof used to make the body surface measurements to provide the EP data 110, or a separate set of electrodes can be used in addition to the set of body surface electrodes. The interval selector 116 can select the sets of beat intervals manually in response to a user input to a user interface 126, such as through a user device 128 (e.g., mouse, keyboard, touchscreen interface, gesture interface or the like). Alternatively, or additionally, the interval selector 116 can be programmed to select the sets of beats automatically.

For example, each of the selected beat intervals includes a set of EP signals associated with a respective beat interval, such as VT beats (e.g., monomorphic VT beats) or other EP signals having a desired recognizable morphology that can be detected. For example, the interval selector 116 is programmed to identify VT beat intervals segments by specifying start and stop time values for the identified VT beats. In other examples, the interval selector is programmed to identify the EP signals as one or more other types of repeating beat intervals, such as atrial tachycardia (AT) beats, sinus rhythm beats and paced beats from various pacing locations (e.g., measured for a VT patient). The EP signals for each respective location across the surface of interest for each of the identified beat intervals can be stored in memory. For example, the EP signals in each set of beat intervals can be stored to represent a plurality of points over a given time window for each respective interval (e.g., such as according to the sample rate at which the signals are acquired or temporal resolution that such signals are derived) or represent a continuous waveform over the given time window.

As a further example, the interval selector 116 can be programmed to select the sets of beat intervals using a matching template having the desired recognizable morphology. For example, the interval selector 116 is programmed to detect respective cardiac beat intervals (e.g., heart beats), such as by identifying an identifiable feature. As an example, the interval selector 116 includes an R-peak detector programmed to identify the R-peak of each of the EP signals (e.g., during a VT storm), and a template window can be defined by the waveform segment between a pair of adjacent points (e.g., middle points or a region of multiple points) between the identified R-peaks. In other examples, the interval selector 116 can use a different morphological feature of the EP signals. The interval selector 116 can employ a moving window to select each set of beats from the EP signals. The interval selector 116 can thus apply the template to detect and identify the beginning and end points of respective beat intervals for grouping EP signals at each of the locations across the surface of interest measured over respective time intervals. The same or different templates can be used at each of the respective locations across the surface of interest. The EP signals in each set of selected beat intervals can be aligned temporally with each other, such as by aligning the R-peaks (or other designated signal feature) of each interval.

The interval selector 116 can implement such template matching and alignment using cross-correlation, correlation coefficient or another method to provide a measure (e.g., a matching value or score) representative of how close each beat interval matches the template. For example, the interval selector 116 is configured to group the selected beat intervals based on the template matching results. The selection and grouping of beat intervals can be based on the matching value, such that those identified EP signals at locations across the surface of interest determined to match the template for a given beat interval better (e.g., having a matching value that exceeds a matching threshold) are selected for inclusion in the given beat interval for the respective location. In another example, the interval selector 116 can be programmed to implement a clustering algorithm (e.g. K-mean clustering algorithm) to generate the sets of beat intervals for selected locations across the surface of interest.

The average calculator 118 is programmed to compute an average (e.g., mean) of each of the selected beat intervals for each of the respective locations. For example, the average calculator 118 averages the signals in each respective set of beat intervals, which have been aligned (as described above), based on the respective cardiac electrophysiological signals in the set of beat intervals selected (by interval selector 116) for each of the respective locations.

As an example, assume that the interval selector 116 has identified a number **M** of matching beats, in which each matching beat exists for a set of locations **L** across the surface of interest and for **T** samples in time. The set of locations **L** can correspond to a set of sensor channels determined (e.g., by a channel integrity function of the mapping system 102) to be good channels. As an example, the channel integrity function can be programmed to determine channel integrity for respective sensors located at the respective locations **L** across the surface of interest, such as according to any of the approaches described in U.S. Patent No. 9,977,060 and/or U.S. Patent No. 10,874,318. For example, the EP signals **S** in the selected set of beat intervals for each location **L** across the surface of interest can be expressed as follows: $S \left(i, j, k\right) , i = 1 . . M , j = 1 . . L , k = 1 . . T$ Eq. 1 The average calculator 118 can be programmed to compute the mean **SM** of the signals **S** in each set of **M** beats at each of the locations **L** over time **T** within a beat, such as according to the following: $SM \left(j, k\right) = \frac{{\sum }_{i = 0}^{M} S \left(i, j, k\right)}{M} , j = 1 .. L , k = 1 .. T$ Eq. 2 The average calculator 118 can store each computed mean beat interval in memory for each of the locations across the surface of interest.

The difference calculator 120 is programmed to calculate a difference between each of the EP signals in each respective set of beat intervals and the average beat interval for the respective set. The differences computed for a given beat interval can provide a difference waveform (a beat interval) for each location **L** across the surface of interest. A set of difference values (e.g., difference waveforms) for each respective beat interval for EP signals at **L** locations across the surface of interest can be computed and stored in memory. The difference calculator 120 can be programmed to compute the difference value by calculating a standard deviation for each respective location based on the set of beat intervals selected for each of the respective locations.

As an example, the difference calculator 120 is programmed to compute the **SS** standard deviation based on a difference between the signals **S** and the mean **SM** across the **M** beats, which can be expressed as follows: $SS \left(j, k\right) = \sqrt{\frac{1}{M} {\sum }_{i = 0}^{M} {\left[S\left(i, j, k\right)-SM\left(j, k\right)\right]}^{2}} , j = 1 .. L , k = 1 .. T$ Eq. 3

The variation calculator 122 is programmed to compute a measure of variation for each of the respective locations across the surface of interest based on the average beat interval and the difference determined for each of the respective locations. Because the variations are associated with the respective locations across the surface of interest, the variation calculator 122 provides a variation map across the surface of interest. The variation calculator 122 can be programmed to compute a variation map **VM** to provide an indication of variation in electrical activity for each of the locations **L** across the surface of interest. For example, the variation calculator 122 is programmed to compute the variation map **VM** as a quotient of the standard deviation **SS** and a magnitude of the mean (e.g., average) beat interval computed for each set of beat intervals. The computation thus operates to scale the standard deviation **SS** in Eq. 3 by the magnitude of the mean signal **SM** (e.g., by taking the absolute value thereof). As an example, the variation calculator 122 can be programmed to compute values in the variation map such as follows: $VM \left(j\right) = \frac{{\sum }_{k = 1}^{T} SS \left(j, k\right)}{{\sum }_{k = 1}^{T} \left|SM\left(j, k\right)\right|} , j = 1 .. L$ Eq. 4 In other examples, the variation calculator 122 can be programmed to implement approaches to calculate values in the variation map.

The mapping system 102 also includes an output generator 132 that is programmed to generate the output data 104, such as to render one or more graphical maps 106 or other information on the display 108. The output generator 132 thus can be programmed to provide one or more graphical maps 106 based on the functions 116, 118, 120, and 122 implemented by the data analysis code 114 to graphically visualize respective variation maps 106 on one or more surfaces of interest. The output generator 132 can generate the output data to provide each respective map 106 in response to a user input via a user interface 126. As disclosed herein, the surface of interest may be the body surface (e.g., outer surface of the thorax), an epicardial surface, an endocardial surface, or a combination of one or more epicardial or endocardial surfaces. Alternatively, or additionally, the surface of interest can be a virtual cardiac envelope, such as a surface residing between the center of a patient's heart and the body surface where the electrodes are positioned. The surface of interest may encompass more than one chamber of the heart. The variation map can be computed directly on the surface of interest or it can be computed on another surface (e.g., the outer surface of the patient's body) and projected on the surface of interest, such as described herein. The surface of interest and view being rendered on the display 108 can be set by the output generator 132 in response to a user input instruction via the user interface 126.

For example, the output generator 132 renders a given variation map as a panoramic graphical representation in which values of the variation map are represented using a color or other scale to describe the variation of EP signals (e.g., electrical potentials) across the surface of interest. Also, a variation map can represent variation in other EP information across the surface of interest derived from the EP data 110 or from another variation map. The variation map can be used to identify targets (e.g., ablation targets), individually or in conjunction with other forms of guidance, such as described herein. The output generator 132 can produce output data 104 that includes a number of one or more graphical variation maps 106 (concurrently or sequentially), such as automatically or in response to a user input through the user interface 126.

As an example, where the surface of interest is the outer surface of a patient's body, and the EP data 118 includes EP signals measured non-invasively by body surface sensor electrodes over one or more time intervals, the data analysis code 114 can be programmed to provide a body surface variation map, in which the variation is computed at respective locations for some or all the of the sensor electrodes (e.g., those locations with good channels). The body surface variation map can be projected onto a cardiac envelope, such as through an inverse reconstruction method implemented by the reconstruction engine 130 and based on the geometry data 112. In other examples, the reconstruction engine 130 can reconstruct the EP signals onto a cardiac envelope and invoke the data analysis code 114 to compute a variation map on the cardiac envelope.

As a further example, the reconstruction engine 130 is programmed to compute the reconstructed signals (e.g., electrical potentials) on the surface of interest by executing machine-readable instructions (e.g., an algorithm) to reconstruct electrical signals spatially and temporally on to the surface of interest based on the EP data 110 and the geometry data 112. For example, the reconstruction engine 130 is configured to compute respective reconstructed electrophysiological signals for a plurality of cardiac nodes spatially distributed over surface of interest based on the EP data 110 measured non-invasively over one or more time intervals. In some examples, the number of cardiac nodes can be greater than 1,000 or 2,000 or more depending upon a desired resolution and the size of the surface of interest. The locations of such cardiac nodes can be stored as part of the geometry data 112.

In some examples, the geometry data 112 includes three-dimensional spatial information representing the surface (or surfaces) of interest describing a surface having locations (e.g. nodes) on to which reconstructed signals are computed (by engine 130) co-registered with respective locations where electrophysiological measurements are made (e.g., by the body surface electrodes). The reconstruction engine 130 can calculate the reconstructed electrical signals on the surface of interest for one or more surfaces of interest over one or more time intervals. The time interval(s) may be selected through the user interface 126 in response to a user input entered by the user device 128 and/or be selected by the interval selector 116.

As a further example, the reconstruction engine 130 includes code programmed to solve the inverse problem for computing reconstructed electrical signals on the surface of interest. In an example, the reconstruction engine 130 is programmed to implement MFS electrocardiographic imaging similar to that disclosed in U.S. patent No. 7,983,743. Other useful examples of inverse algorithms that can be implemented by the reconstruction engine 130 to reconstruct include the boundary element method (BEM), such as disclosed in U.S. patent Nos. 6,772,004, and 9,980,660. The reconstruction engine 130 further may employ a regularization technique (e.g., Tikhonov regularization) to estimate values for the reconstructed electrical signals on the surface of interest. The reconstruction engine 130 can implement any of a variety of approaches to solve the inverse problem for reconstructing the electrophysiological signals onto one or more surfaces of interest.

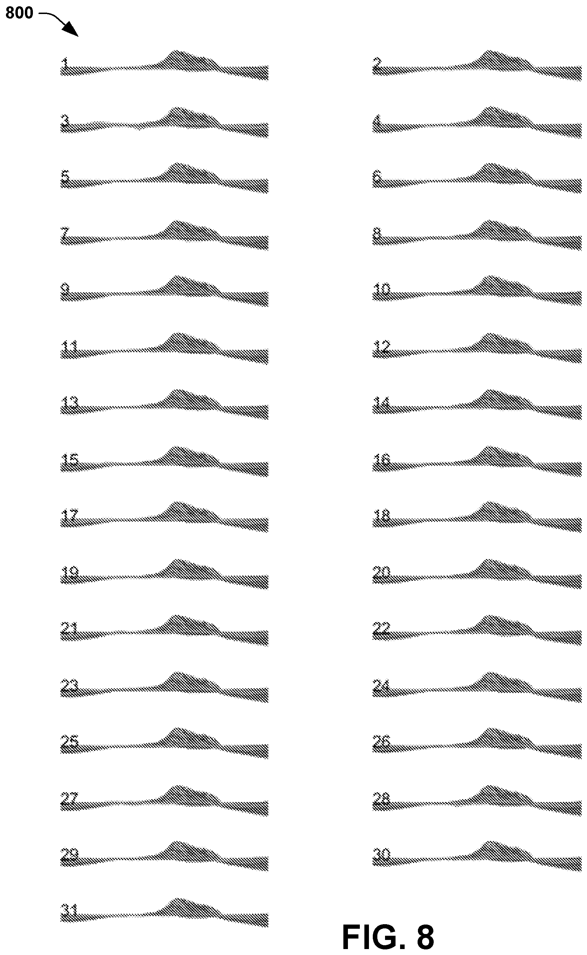

In some examples, the mapping system includes a gating function 134 programmed to implement gating of the EP signals (e.g., in the EP data 110 and/or reconstructed EP signals) based on physiological data 124. For example, the interval selector 116 is programmed to identify a set of respective beat intervals for each of the respective locations across the surface that occur within a respective phase (e.g., at or overlap in time) of a physiological function of the patient during the time when the EP data is acquired. The gating function can identify one or more phases of the physiological function in the physiological data 124, such as automatically or in response to a user input selecting which one or more phases of the function (see, e.g., FIG. 8). For example, the physiological data 124 includes information representative of a waveform describing the physiological function over time (e.g., the same one or more time intervals represented in the EP data 110). The physiological function can include respiration or movement of the patient during the acquisition of the EP data. In an example, the gating function 134 is programmed to provide gating data to identify one or more phases of physiological function having reduced patient movement (e.g., at the end of an inhalation or exhalation). The gating function 134 can use other phases of the respiratory cycle in other examples, as well as different physiological functions. The interval selector 116 can select the set of beat intervals for each of the respective locations based on the gating data (from gating function 134) so that the selected set of beat intervals at each location occur within or overlap with the identified phase(s).

In examples where more than one phase of a physiological condition or multiple conditions are used, the interval selector 116 can be programmed to group beat intervals into multiple phase groupings for each of the respective locations based on which phase each of the respective beat intervals resides as a function of time (e.g., temporally). Each of the other data analysis functions, including the average calculator 118, the difference calculator 120 and the variation calculator, thus are programmed to compute respective information for each of the respective phase groupings of beat intervals at respective locations. For example, instances of the average calculator code 118, the difference calculator code 120 and the variation calculator code 122 can be invoked separately to determine a respective measure of variation across the surface of interest for each of the phase groupings. Accordingly, separate variation maps can be generated for each of the respective phase groupings.

FIG. 2 is a block diagram that illustrates another example system 200 that includes a mapping system 102 as well as a therapy system 202, such as for applying a therapy or other treatment based on one or more maps that can be generated by the mapping system. The system 200 can implement various hardware and software components shown and described with respect to FIG. 1. Accordingly, the description of FIG. 2 also refers to FIG. 1. FIG. 2 focuses on additional features relating to EP signal measurements as well as invasive treatment functions and related features that can be implemented.

The system 200 includes an arrangement of body surface electrodes 204, which are coupled to a signal measurement system 206. For example, each of the electrodes 204 is coupled to the signal measurement system 206 through a respective electrically conductive channel (e.g., including electrically insulated wires and/or traces, optical fibers or wireless leads) to communicate electrophysiological signals measured from the patient's body. The electrically conductive channels for the electrodes 204 can include an arrangement of connectors configured to couple to respective connectors (e.g., male and female connectors) of the measurement system 206.

The body surface electrodes 204 include an arrangement of multiple electrodes (e.g., 20-300 sensor electrodes or any permutation thereof) distributed across an outer surface of the patient's body 208. In an example, the body surface electrodes 204 are distributed completely around the thorax, such as can be mounted to a wearable garment (e.g., vest) in which each of the electrodes has a known location in a given coordinate system. As an example, body surface electrodes 204 can be implemented as a non-invasive type of sensor apparatus as disclosed in U.S. Patent No. 9,655,561, entitled Multi-Layered Sensor Apparatus. Other configurations and numbers of body surface electrodes 204 could be utilized in other examples, such as on patch electrodes or an arrangement of individually placed sensing electrodes.

The system 200 also includes an invasive device 210 that includes one or more invasive electrodes. The invasive device 210 can be positioned on or within the patient's heart 212. Each of the electrodes 204 and those on the device 210 can be coupled to the signal measurement system 206 through a respective communications medium to communicate electrophysiological signals measured from the patient's body 208. The measurement system 206 can be configured to measure unipolar, bipolar or a combination of unipolar and bipolar electrophysiological signals. The signal measurement system 206 thus provides the EP data 110 representative electrical activity measured by the electrodes 204 and electrodes of device 210 over time in which the signal for each respective electrode corresponds to respective channel.

The signal measurement system 206 can include signal processing circuitry configured to process electrical signals received by the electrodes 204 as well as from electrodes on the invasive device 210. The signal processing circuitry can be implemented as hardware and/or software, such as including a digital signal processor and other processing circuitry and machine-readable instructions (executable by a processor) configured to remove noise at known frequencies (e.g., baseline noise, power line noise) and convert the received signals into a desired format. Additionally, the mapping system 102 can include additional signal processing function 224 (e.g., program code) programmed to perform further signal processing functions. The signal processing function 224 can include a channel integrity function, such as to remove bad channels and interpolate among adjacent signals to improve accuracy. The signal processing function 224 can also include filtering, such as including low-pass filtering, high-pass filtering (e.g., to remove breathing/baseline noise), band-pass filtering (e.g., to remove known frequency noise such as power line/instrument noise).

Additionally, or alternatively, the invasive device 210 can be implemented as or including an ablation device, such as a radio frequency (RF) ablation probe, a cyroablation device, an irreversible electroporation (IRE) device, a laser ablation device or the like. For example, the invasive device 210 can be a catheter probe that is moveable within the patient's body 208, such that the position of the probe and associated electrode(s) or ablation elements thereof can vary within the patient's body. The therapy system 202 is configured to control application of energy or other treatment applied by the invasive device, which can be in response to a user input and/or automatically responsive to the output data 104.

The mapping system 102 can also include reconstruction engine 130, user interface 126, output generator 132 and data analysis code 114. As described herein, the data analysis code 114 can include instructions (e.g., functions 116, 118, 120, 122 and 134 of FIG. 1) programmed to perform respective analysis functions to ascertain variation in electrical activity across one or more surfaces of interest. The data analysis code 114 thus can be programmed to at least: select a set of beat intervals for respective locations, compute an average/mean for each set of beat intervals, compute a difference between the signals in each respective set of beat intervals and the average of such set, and compute an indication variation at the respective locations across the surface of interest (e.g., one or more variation maps), as described herein.

In FIG. 2, the system 200 also includes a navigation system 214 configured to localize the spatial position of the invasive device 210 and/or other objects that might be positioned on or within the patient's body 208. The spatial position of the invasive device 210 can be stored in memory as location data 216. The location data 216 thus represents a three-dimensional spatial position (e.g., spatial coordinates) of the invasive device 210. The spatial location of the invasive device 210 can be with respect to the patient's body or a coordinate system of the navigation system 214. The location data 216 can also include a time stamp so that the mapping system 102 can programmatically link (e.g., synchronize) to a given time instance of the geometry data, which includes location of the device 210.

Useful examples of the navigation system 214, which can be used to localize the invasive device 210, include the CARTO EP navigation system (commercially available from Biosense-Webster), the ENSITE visualization and navigation technology (commercially available from Abbott), and AcQMap (commercially available from Acutus); although other navigations systems could be used to provide the navigation data representative of the spatial position for the invasive device 210 and associated sensors.

In the example of FIG. 2, the mapping system 102 includes navigation control code 218 and spatial registration code 220. The navigation control code 218 is programmed to interface with and control the navigation system 214. The spatial registration code 220 is programmed to register the spatial location of the invasive device 210 and/or other objects (e.g., sensors) being localized, as described by or derived from the location data 216, with respect to anatomical geometry of the patient's body 208 (e.g., provided in geometry data 112). The registration process can be repeated continually (e.g., based on a localization sample rate) or in response to detecting changes in the location data 216 as the electrode is moved within the patient's body. In some examples, the navigation system 214 can also be programmed to provide the location data 216 to represent the location of one or more of the non-invasive electrodes 204, which are distributed across an outer surface of the patient's body (e.g., on the thorax) 208. The spatial registration code 220 can register the electrode locations represented by location data 216 into a common spatial domain with the patient's body to provide part of the geometry data 112, namely, the locations of some or all of the electrodes 204.

In an example, the navigation control 218 can be programmed to cause the spatial registration code 220 to update the geometry data 112 over time, such as to represent a changing spatial position of the invasive device 210 relative to the patient's body 208, including the heart 212, and/or body surface electrodes 204 (e.g., responsive to. The navigation control 218 can also provide guidance to help a user position the device 210 at or near a target location (e.g., an ablation target) within the patient's body. The target location can be an ablation target site or other region of interest identified in a variation map generated by the data analysis code 114, as described herein. For example, the navigation control code 218 is programmed to compute a distance between the target location and a current position of the invasive device 210, which has been spatially registered by spatial registration code 220 with patient anatomy. The navigation control 218 can be programmed to compute the distance from the graphical map based on a number of pixels along a line connecting the device 210 and the prescribed target location, which can be translated to a physical distance. Alternatively, the navigation control 218 can be programmed to track the distance between the electrode and the target location(s) in a co-registered spatial domain.

As described herein, the output generator 132 configured to generate output data 104 to render one or more graphical maps (e.g., a variation map superimposed on a body surface, such as the torso and/or heart) 106 and/or display processed electrical signals on the display 108 derived from the EP data 110. The mapping system 102 can also provide information in other display formats to provide guidance to the user representative of and/or derived from electrical activity that may be measured by any combination of the electrodes 204 and electrode(s) carried by the device 210. In the context of delivering therapy and/or measuring electrophysiological signals using the invasive device 210, the output generator 132 can provide guidance, such as a graphical, color coded representation of a variation map 324 on the display 326.

As described herein, the variation maps 106 can be used to localize of one or more ablation targets (e.g., one or more spatial regions or points) on the patient's heart 212, such as by graphically differentiating the targets on the surface of interest. In an example, the ablation targets identified in the variation map can be supplied as output data 104 for rendering as graphical variation map 106 and to provide guidance for treating one or more targets, such as for monomorphic VT or other arrhythmogenic conditions. In another example, the variation map can be combined with one or more other types of electroanatomic maps, which can be generated by the mapping system 102, such as a directional activation map, a rotor (or rotational domain) map, an activation map, voltage potential map, slew rate map and the like. The mapping can also be extended to virtually any single beat based maps, such as including on repolarization maps generated on T waves or the like. Thus, variation maps can be computed to display an indication of any EP condition that can be mapped across a number of surfaces.

The data analysis code 114 and/or the output generator 132 can be programmed to combine the variation map with one or more other maps to validate the location of treatment targets. For example, the output generator 132 can be programmed to generate a composite map in the output data 104 for graphical rendering on the display 108. The composite map thus can identify a subset of targets for treatment based on a correlation between the respective maps.

In a further example, the output generator 132 can generate the output data to provide guidance by instructing a user to place the invasive device 210 at one or more prescribed locations (e.g., identified targets) within the patient's body in response to output data 104. The localization of the prescribed target location to the user can further be guided based on the location data 216 generated by the navigation system 214, such as in response to instructions provided by the navigation control 218.

The therapy system 202 can include a control system 222 (e.g., hardware and/or software) configured to control application of a therapy, which can be administered via the device 210 invasively and/or non-invasively. In an example, the therapy system 202 is configured to ablate tissue responsive to control signal from the control system 222. The ablation can be provided as radio frequency ablation, radiation ablation, cryoablation, noninvasive cardiac radioablation, or pulsed-field ablation. The therapy system 202 can be configured to provide other types of treatment in other examples, such as pacing to determine on or more implant sites for an implantable cardioverter-defibrillator. Thus, a user can locate a target site on a cardiac surface (endocardial and/or epicardial), as identified by the guidance provided in the variation map, individually or in combination with other guidance, and perform a desired intervention at the target site, such as an ablation, pacing (e.g., at one or multiple frequencies), delivery of a drug, etc.

After performing the ablation or other treatment, which can be applied at the target site or a therapeutic agent (e.g., cardiac targeted drug delivery), the patient's cardiac electrophysiology can be re-evaluated by acquiring EP data after the ablation. For example, one or more variation maps can be generated based on the post-treatment EP data, such as described herein. The post-treatment variation map(s) can be compared to the previous (e.g., pre-treatment) variation maps, to determine whether the treatment has reduced or eliminated the variation or other identified arrhythmogenic condition (e.g., VT). For example, the data analysis code 114 can be programmed to compute a difference between the pre-treatment variation map and the post-treatment variation map. The data analysis code can be used to provide guidance to a user for subsequent application of treatment at the same or a different target site depending on whether the measure of variation increases or decreases responsive to a given treatment. The subsequent treatment can be th same or a different form of treatment. For example, the therapy system 202 can be used (with guidance provided by the navigation system 214) to apply another treatment at the same target site of the heart in response to determining that the previous treatment has reduced the measure of variation across the surface(s) of interest. Alternatively, the therapy system 202 can be used to apply another treatment at the same or a different target site of the heart in response to determining that the measure of variation across the surface(s) of interest the previous treatment has not changed or increased responsive to the previous treatment. The process can continue until the variation map indicates an acceptable measure of variation across the surface(s) of interest and/or a normal sinus rhythm is achieved.

In view of the structural and functional features described with respect to FIGS. 1 and 2, FIGS. 3 and 4 are flow diagrams showing example methods of generating and using variation maps. While the example methods are shown and described as executing serially, the example methods are not limited by the illustrated order, as some actions could in other examples occur in different orders, multiple times and/or concurrently from that shown and described herein. In an example, the methods can be implemented as machine-readable instructions executed by a processor, such as by a computing apparatus (e.g., a local computer, a cloud-based computer or a computing apparatus that include hardware and/or software distributed between a local premise and a cloud computing architecture). The methods can also be implemented by instructions being executed by one or more processors of the mapping system 102 of FIGS. 1 and 2. In an example, the methods can be implemented by one of the CardioInsight^{®} mapping products (e.g., mapping vest and workstation) available from Medtronic of Minneapolis, Minnesota USA. In other examples, other cardiac mapping systems can be programmed to implement the method 300 (e.g., the ENSITE^{®} cardiac mapping systems from Abbott or the CARTO^{®} system from Biosense Webster). That is, methods disclosed herein are applicable to nearly any form or configuration of EP mapping, recording and/or navigation system.

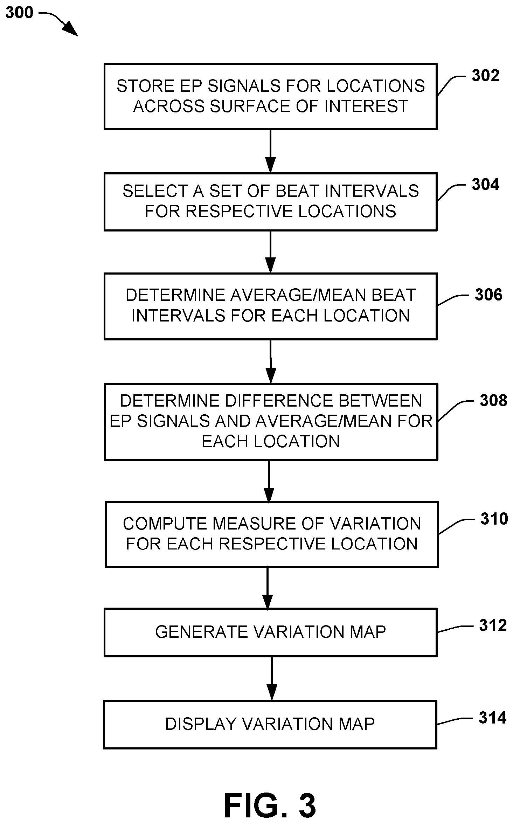

FIG. 3 is a flow diagram showing an example method 300 of generating variation maps. The method 300 begins at 302 in which EP signals for locations across the surface of interest are stored in memory. The signals can represent real-time EP signals being acquired over time intraoperatively (e.g., during an EP study or treatment procedure) or be acquired over a prior time period (e.g., for off-line analysis). The surface of interest thus can be the outer surface of a patient's body and/or a cardiac surface (e.g., an epicardial surface, endocardial surface or a virtual cardiac surface within the patient's body). The EP signals thus can be measured directly at the locations on the surface of interest (e.g., by sensing electrodes), non-invasively and/or invasively. Additionally, or alternatively, the EP signal can be reconstructed or projected onto the surface mathematically (e.g., by reconstructing signals onto the cardiac surface from EP signals measured on the body surface).

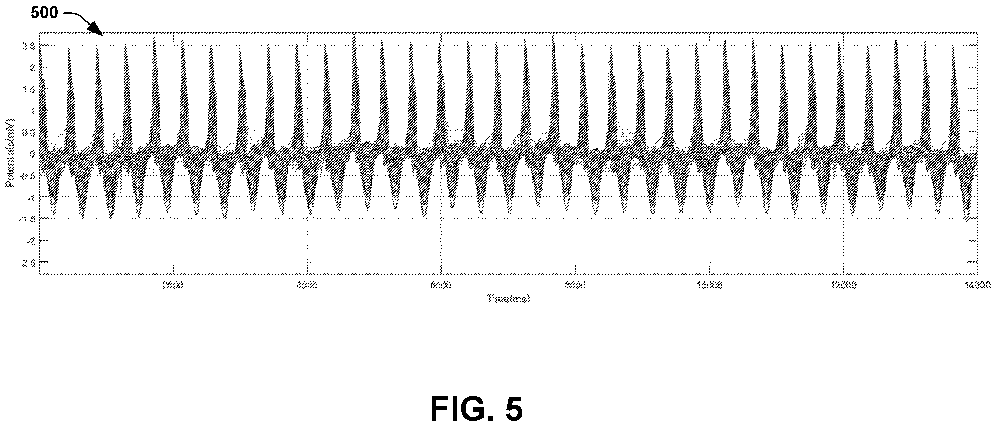

FIG. 5 is signal diagram showing example EP signals (e.g., electrograms) 500 measured by electrodes (e.g., electrodes 204 of FIG. 2) distributed across an outer surface of a patient's body. The EP signals 500 include waveforms having a plurality of beat intervals during a VT storm. The EP signals thus show one type of EP signals that be stored in memory (at 302).

At 304, the method includes selecting (e.g., by interval selector 116) a set of beat intervals (from the signals at 302) for the respective locations across the surface of interest. For example, the selected beat intervals can include a set of VT beats (e.g., monomorphic VT beats) or other signals having a desired recognizable morphology that can be detected. As described herein, for example, the selected set of beat intervals are selected (e.g., by interval selector 116) using a matching template adapted to recognize a desired waveform morphology and identify the beginning and end points of respective beat intervals. A morphological feature of the beat intervals (e.g., R-peak) can be used to align the EP signals in each respective beat interval. Bad channels or sensors can be omitted from the sets of EP signals so that the beat intervals selected (at 304) include only channels that have been determined to be good channels.

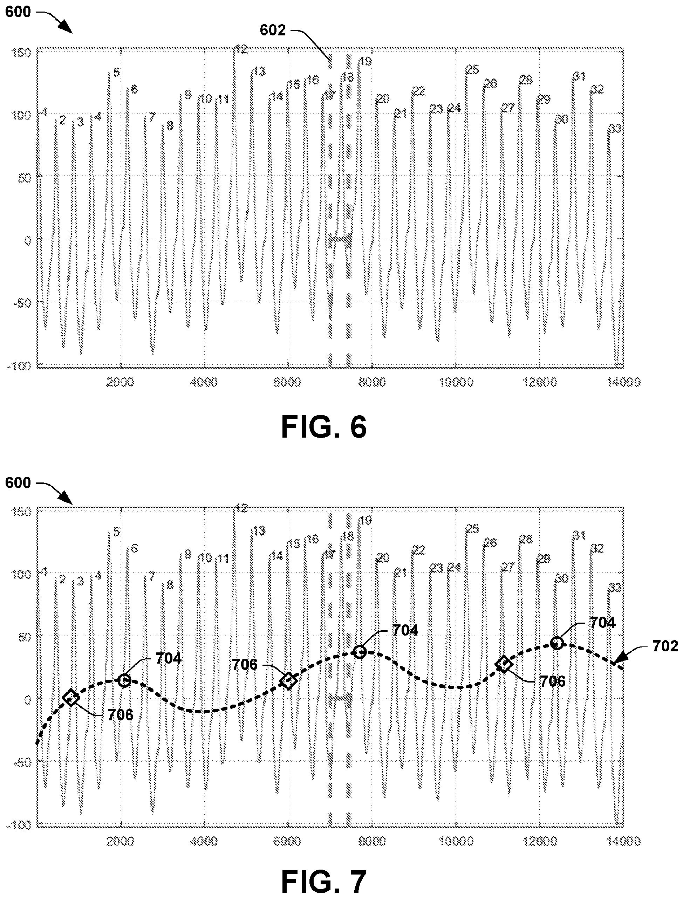

FIGS. 6 and 7 are signal diagrams showing an example of an EP signal 600 that represents electrical activity at a given location across the surface of interest (e.g., an outer surface of a patient's body), such as one of the signals shown in FIG. 5. In the examples of FIGS. 6 and 7, 33 beat intervals are shown for the given location over a continuous time interval. Similar signals are provided for each other location across the surface(s) of interest. Calipers are shown schematically at 602 for identifying the start and end of a respective beat interval (interval #18). The beginning and end of each selected beat can be identified similarly, such as described herein.

FIG. 7 also shows another waveform 702 superimposed on the EP signal 600. For example, the waveform 702 represents another physiological function, such as a patient's respiratory cycle, which can be used at 304 for selecting beats of the signal 600 into respective beat intervals. For example, the set of beat intervals can be selected (at 304; e.g., by interval selector 116) from the EP signal 600 based on the gating data (from gating function 134) so that the beat intervals in a respective set of beat intervals each reside within or overlap with one or more identified phases, shown at 704 and 706. For example, the phase 704 represents a peak of the physiological function and the phase 706 represents a rising midpoint of the physiological function. Accordingly, beats #6, #19 and #30 can be selected from the signal 600 at 304 (along with similarly aligned beats at other respective locations across the surface of interest) for further processing to generate a first phase-specific variation map, as described herein. Similarly, beats #3, #15 and #27 can be selected from the signal 600 at 304 (along with similarly aligned beats at other respective locations across the surface of interest) for further processing to generate a second phase-specific variation map, as described herein. In some examples, beats for more than one phase of interest can be combined to provide multiple sets of respective beats for use in generating a variation map. Other numbers and types of physiological functions can be used in other examples for generating corresponding maps.

FIG. 8 is a signal diagram showing 800 signal waveforms in multiple sets of beat intervals, which can be selected (e.g., by interval selector 116) in the method 300 at 304. While the example of FIG. 8 has 31 sets of beat intervals, other numbers of beat intervals can be selected, which can be less than or greater than 31 beats (e.g., three or more beats). As described with respect to FIG. 7, the selection of sets of beat intervals further can be based on one or more phases of a physiological function. As shown in FIG. 8, each of the EP signals for each beat are aligned between respective start and end points, such as can be selected for each beat as a point (or a region of multiple points) in time between pairs of adjacent R-peaks, such as shown at 602 in FIG. 6.

At 306, an average (e.g., mean) beat interval can be calculated (e.g., by average calculator 118) for each of the beat intervals based on the respective EP signals in each set of beat intervals selected (at 304). The average or mean beat interval can be stored in memory for each of the locations across the surface of interest. For example, with reference back to the example of FIG. 8, the signals in each respective beat interval can be averaged together to provide 31 average beats. In an example, the average computed at 306 can be determined according to Eq. 2.

At 308, a difference can be calculated (e.g., by difference calculator 120) between each EP signal in a respective set of beat intervals and the average or mean beat interval for the beat interval. A set of difference values for each beat interval and respective locations across the surface of interest can be computed and stored in memory. The difference value can be determined by calculating a standard deviation for each respective location based on the set of beat intervals selected for each of the respective locations. In an example, the difference computed at 308 can be determined according to Eq. 3.

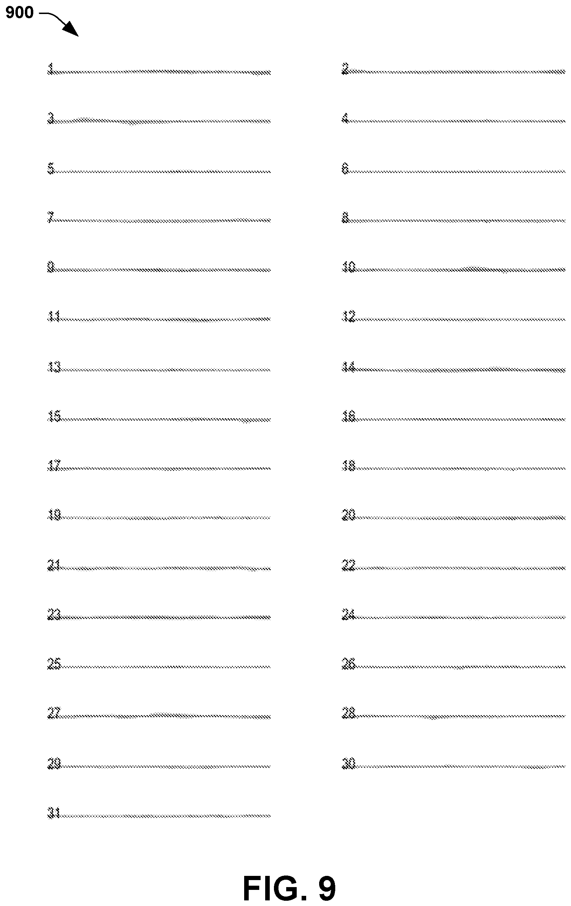

FIG. 9 is a signal diagram showing difference signal waveforms 900 for respective sets of beat intervals, which can be determined (e.g., by difference calculator 120) in the method 300 at 308. For example, the difference signal waveforms 900 are shown for 31 beat intervals, such as computed from the beat intervals in FIG. 8 and corresponding averages thereof determined at 306.

At 310, a measure of variation is computed for each of the respective locations across the surface(s) of interest. For example, the variation calculator 122 is programmed to compute the measure of variation based on the mean beat interval and the differences determined for each of the beat intervals and respective locations across the surface of interest. The mean, difference and measure of variation that are determined can each represent a plurality of points over a time window (e.g., over a beat interval) or represent a continuous waveform over the time window. The systems and methods can also generate a variation map across the surface of interest based on the measure of variation computed for each of the respective locations. In examples where the surface of interest is the outer surface of a patient's body, the variation map can be projected onto a cardiac envelope, such as by reconstructing signals onto the cardiac surface from EP signals measured on the body surface. In an example, the measure of variation computed at 310 can be calculated according to Eq. 4.

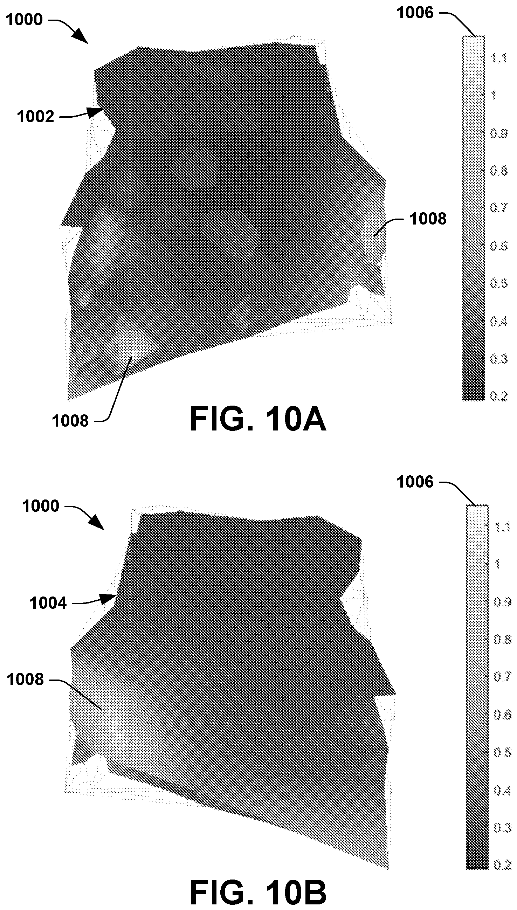

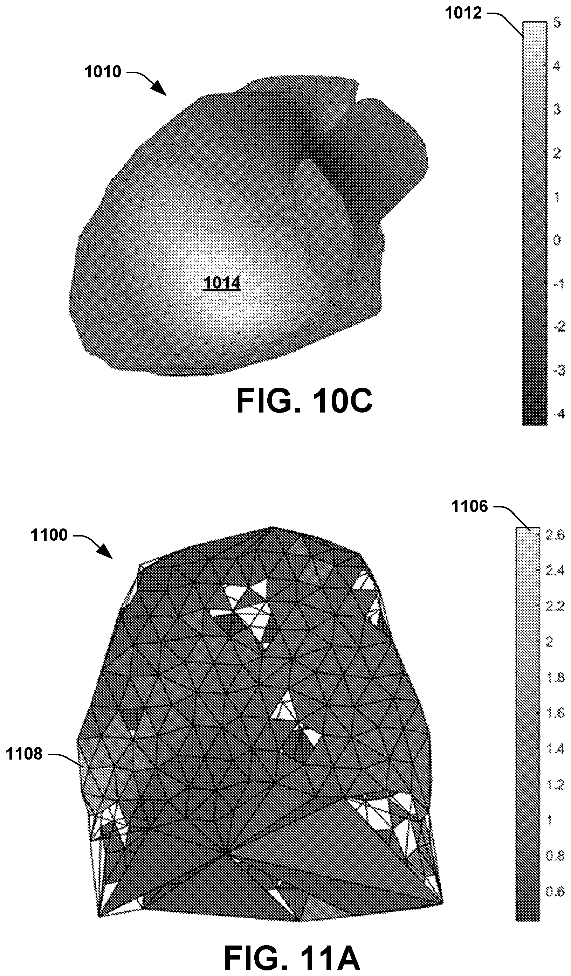

At 312, a variation map can be generated. The variation map describes electrical variation across the surface of interest based on the EP signals in the selected beat intervals. At 314, the variation map (generated at 312) is displayed. For example, output generator 132 can generate output data 104 to render one or more graphical variation maps, such as shown in FIGS. 10A, 10B and 10C.

FIGS. 10A and 10B depict an example of a graphical variation map 1000 based on measures of variation computed at 310 across a body surface (e.g., torso) of patient, such as the map 106 that can be rendered on display 108. FIGS. 10A and 10B show posterior and anterior views 1002 and 1004, respectively, for a variation map of the torso. The map includes a scale (e.g., a normalized scale), shown at 1006, such as to visualize electroanatomical variation across the body surface. Regions exhibiting higher amounts of variation are shown at 1008. FIG. 10C depicts an example of another graphical variation map 1010 to visualize electroanatomical variation across a cardiac surface (e.g., epicardial surface). Similar to the body surface map 1000, the cardiac map 1010 includes a scale, shown at 1012, to visualize electro anatomical variation across the surface, in which regions exhibiting higher amounts of variation is shown at 1014. For example, the map 1010 can be generated by projecting the body surface variation map (from FIGS. 10A and 10B) onto the cardiac surface, such as through an inverse reconstruction (e.g., by reconstruction engine 130 based on geometry data 112). Alternatively, the map 1010 can be generated by applying the method 300 to reconstructed EP signals, which have been reconstructed onto the cardiac surface (e.g., by reconstruction engine 130 based on geometry data 112). Other types of maps including different variation maps can also be generated on one or more surfaces according to the method 300.

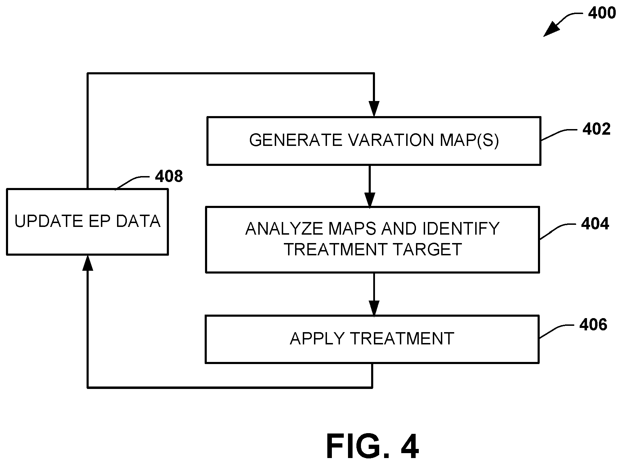

FIG. 4 is a flow diagram of an example method 400 of treating a patient. The method 400 can be used in combination with the method 300 of FIG. 3. For example, at 402, the method 400 includes generating one or more variation maps. Each variation map can be generated according to the systems and methods described herein. At 404, the one or more maps are analyzed (e.g., manually and/or by data analysis code 114) to identify one or more treatment targets. At 406, treatment is applied. As described herein, the treatment can include ablation, pacing or another treatment modality. After the treatment at 406, the method proceeds to 408, in which EP data is updated to provide post-treatment EP data. From 408, the method returns to 402 and the method can be repeated, if desired, based on the post-treatment EP data. Also, the analysis at 404 can include an analysis of a number of variation and/or other maps that have been generated during the method 400, longitudinally at different phases of treatment.

While examples herein have focused on generating variation maps based on measured electrical potentials, the systems and methods herein can be configured to generate variation maps on one or more surfaces of interest based on other physiological information, such as based on other maps representative of electrophysiological information derived from the electrical potentials across the surface(s) of interest (e.g., including from directional activation map, voltage map, slew rate map, repolarization map etc.). As an example, activation time and/or directional activation can be derived from EP data representing EP signals at locations (e.g., points or nodes) across a surface of interest. The systems 100, 200 and/or method 300 can process the activation and/or directional activation time data, as described herein, to generate a measure of variation in the activation time and/or directional activation time across the surface interest.

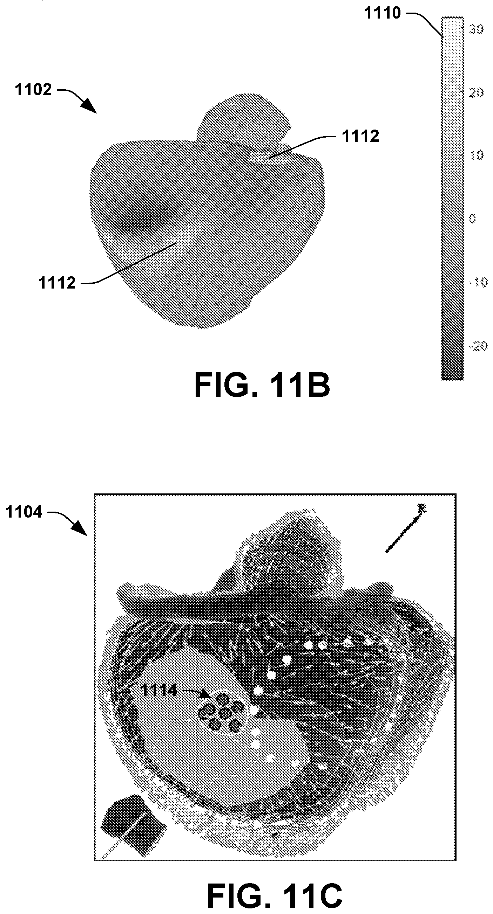

FIGS. 11A, 11B and 11C depict examples of some different types of variation maps 1100, 1102 and 1104 that can be generated using the systems or methods described herein, such as based on EP signals measured non-invasively from a patient's body surface. Specifically, the map 1100 of FIG. 1100 is variation map across a patient's torso representing variation according to a scale 1106, in which a high variation region is shown at 1108. In the example of FIG. 11A, white areas can identify bad channels excluded from variation map. The map 1102 of FIG. 11B is an example directional activation time variation map across the heart. The map 1102 also shows variation on the cardiac surface according to a scale 1110, in which regions of increased variation are shown at 1112. The map 1104 of FIG. 11C is an example of activation flow variation map across the heart. The map 1104 also shows variation on the cardiac surface, in which regions of increased variation are shown at 1114.

It should be understood that various aspects disclosed herein may be combined in different combinations than the combinations specifically presented in the description and accompanying drawings. It should also be understood that, depending on the example, certain acts or events of any one of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device. In this disclosure, the term "based on" means based at least in part on. Also, in this disclosure the term "responsive to" and variants thereof mean responsive at least in part to.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any one of the foregoing structure or any other physical structure suitable for implementation of the described techniques. Also, the techniques could be fully implemented in one or more circuits or logic elements.

## Claims

1. A system (102), comprising:
one or more non-transitory computer-readable media to store data and machine-readable instructions, the data including electrophysiological data representative of cardiac electrophysiological signals at respective locations across a surface of interest; and
a processing unit (114) to access the non-transitory computer-readable media and execute the machine-readable instructions, the machine-readable instructions including: interval selector (116) code programmed to select multiple sets of beat intervals, in which each set of beat intervals includes respective cardiac electrophysiological signals at respective locations across the surface of interest for the respective beat interval;
average calculator (118) code programed to determine an average beat interval for each set of beat intervals based on the respective cardiac electrophysiological signals in the respective set of beat intervals;
difference calculator (120) code programmed to determine, for each set of beat intervals, a difference between each of the respective cardiac electrophysiological signals and the average beat interval for each respective set of beat intervals; and
variation calculator (122) code programmed to compute a measure of variation for each of the respective locations across the surface of interest based on the average beat interval and the differences determined for the respective beat intervals across the surface of interest, in which a variation map across the surface of interest is generated based on the measure of variation computed for each of the respective locations.

2. The system of claim 1, wherein:
the difference calculator code is programmed to compute a standard deviation across the beat intervals based on the respective cardiac electrophysiological signals and average determined for each set of beat intervals, and
the variation calculator code is programmed to compute the measure of variation for each of the respective locations based on a quotient of the standard deviation and a magnitude of the average beat interval computed for each of the respective locations.

3. The system according to any one of claims 1 or 2, wherein the interval selector code is further programmed to:
match cardiac electrophysiological signals for each respective location to an interval template window to provide each of the sets of beat intervals; and
group respective cardiac electrophysiological signals for each set of the beat intervals based on a matching value determined for each respective set of beat intervals.

4. The system according to any one of claims 1 or 2, wherein the interval selector code is further programmed to align each of the respective cardiac electrophysiological signals in each set of beat intervals based on a signal morphology feature.

5. The system of claim 4, wherein the respective cardiac electrophysiological signals are representative of one of a ventricular tachycardia, a normal sinus rhythm or an atrial tachycardia, and the signal morphology feature is a point or region located between adjacent R-peaks of the respective cardiac electrophysiological signals.

6. The system according to any one of claims 1 or 2, wherein the interval selector code is further programmed to:
identify a plurality of beat intervals that occur at, or overlap with, a respective phase of a physiological function having multiple phases, in which each of the multiple sets of beat intervals are selected from the identified plurality of beat intervals.

7. The system of claim 6, wherein the interval selector code is further programmed to group subsets of the beat intervals into respective phase groupings based on which phase of the multiple phases of the physiological function the respective beat intervals reside within temporally, and
wherein an instance of the average calculator code, the difference calculator code and the variation calculator code are invoked separately to determine a respective measure of variation across the surface of interest for each of the phase groupings.

8. The system according to any one of claims 1-7, wherein the surface of interest is an outer surface of a patient's body, and the variation map is a body surface variation map across the outer surface.

9. The system of claim 8, further comprising reconstruction code programmed to project the variation map onto a cardiac surface of interest to provide a cardiac surface variation map.

10. The system according to any one of claims 1-7, wherein the surface of interest is cardiac surface of interest within a patient's body, the respective cardiac electrophysiological signals are reconstructed electrophysiological signals at respective locations across the cardiac surface of interest based on electrophysiological signals measured non-invasively across a surface of the patient's body over one or more time intervals and geometry data, and the variation calculator code is programmed to generate a cardiac surface variation map across the cardiac surface of interest.

11. The system according to any one of claims 1-7, further comprising:
an arrangement of body surface electrodes adapted to measure the cardiac electrophysiological signals non-invasively from an outer surface of a patient's body, the instructions further comprising reconstruction code programmed to reconstruct electrophysiological signals on locations distributed across the surface of interest within the patient's body to provide the electrophysiological data representative of cardiac electrophysiological signals at respective locations across the surface of interest based on the measured cardiac electrophysiological signals and geometry data.

12. The system according to any one of claims 1-11, wherein the instructions include data analysis code programmed to:
identify at least one target site based on the variation map;
provide guidance for applying a treatment at the target.

13. The system of claim 12, wherein:
the treatment is a first treatment, and after the first treatment has been applied, updated cardiac electrophysiological signals are provided across the surface of interest,
each of the interval selector code, the average calculator code, the difference calculator code and the variation calculator code programmed to determine an updated measure of variation across the surface of interest based on the updated electrical physiological signals, and
the data analysis code is further programmed to provide guidance for applying a second treatment at the same target location or a different target location based on the updated measure of variation or based on a difference between the updated measure of variation and the measure of variation before applying the first treatment.

14. The system according to any one of claims 1-13, further comprising output generator code programmed to generate output data to display a graphical representation of the variation map.

## Patentansprüche

1. System (102), umfassend:
ein oder mehrere nicht-transitorische computerlesbare Medien zum Speichern von Daten und maschinenlesbaren Anweisungen, wobei die Daten elektrophysiologische Daten beinhalten, die elektrophysiologische Herzsignale an jeweiligen Orten über eine interessierende Oberfläche hinweg repräsentieren; und
eine Verarbeitungseinheit (114) zum Zugreifen auf die nicht-transitorischen computerlesbaren Medien und zum Ausführen der maschinenlesbaren Anweisungen, wobei die maschinenlesbaren Anweisungen Folgendes beinhalten:
Intervallauswahlcode (116), der dazu programmiert ist, mehrere Sätze von Schlagintervallen auszuwählen, wobei jeder Satz von Schlagintervallen jeweilige elektrophysiologische Herzsignale an jeweiligen Orten über die interessierende Oberfläche hinweg für das jeweilige Schlagintervall beinhaltet;
Durchschnittsberechnungscode (118), der dazu programmiert ist, ein durchschnittliches Schlagintervall für jeden Satz von Schlagintervallen basierend auf den jeweiligen elektrophysiologischen Herzsignalen in dem jeweiligen Satz von Schlagintervallen zu bestimmen;
Differenzberechnungscode (120), der dazu programmiert ist, für jeden Satz von Schlagintervallen eine Differenz zwischen jedem der betreffenden elektrophysiologischen Herzsignale und dem durchschnittlichen Schlagintervall für jeden betreffenden Satz von Schlagintervallen zu bestimmen; und
Variationsberechnungscode (122), der dazu programmiert ist, ein Variationsmaß für jeden der betreffenden Orte über die interessierende Oberfläche hinweg basierend auf dem durchschnittlichen Schlagintervall und den Differenzen, die für die jeweiligen Schlagintervalle über die interessierende Oberfläche hinweg bestimmt werden, zu berechnen, wobei eine Variationskarte über die interessierende Oberfläche hinweg basierend auf dem Variationsmaß erzeugt wird, das für jeden der betreffenden Orte berechnet wird.

2. System nach Anspruch 1, wobei:
der Differenzberechnungscode dazu programmiert ist, eine Standardabweichung über die Schlagintervalle hinweg basierend auf den jeweiligen elektrophysiologischen Herzsignalen und dem Mittelwert, der für jeden Satz von Schlagintervallen bestimmt wird, zu berechnen, und
der Variationsberechnungscode dazu programmiert ist, das Variationsmaß für jeden der betreffenden Orte basierend auf einem Quotienten aus der Standardabweichung und einer Größe des durchschnittlichen Schlagintervalls, das für jeden der betreffenden Orte berechnet wird, zu berechnen.

3. System nach einem der Ansprüche 1 oder 2, wobei der Intervallauswahlcode ferner dazu programmiert ist:
elektrophysiologische Herzsignale für jeden betreffenden Ort mit einem Intervallvorlagenfenster abzugleichen, um jeden der Sätze von Schlagintervallen bereitzustellen; und
jeweilige elektrophysiologische Herzsignale für jeden Satz der Schlagintervalle basierend auf einem Übereinstimmungswert, der für jeden betreffenden Satz von Schlagintervallen bestimmt wird, zu gruppieren.

4. System nach einem der Ansprüche 1 oder 2, wobei der Intervallauswahlcode ferner dazu programmiert ist, jedes der betreffenden elektrophysiologischen Herzsignale in jedem Satz von Schlagintervallen basierend auf einem Signalmorphologiemerkmal auszurichten.

5. System nach Anspruch 4, wobei die jeweiligen elektrophysiologischen Herzsignale eines von einer ventrikulären Tachykardie, einem normalen Sinusrhythmus oder einer atrialen Tachykardie repräsentieren und das Signalmorphologiemerkmal ein Punkt oder ein Bereich ist, der sich zwischen benachbarten R-Zacken der jeweiligen elektrophysiologischen Herzsignale befindet.

6. System nach einem der Ansprüche 1 oder 2, wobei der Intervallauswahlcode ferner dazu programmiert ist:
eine Mehrzahl von Schlagintervallen zu identifizieren, die in einer jeweiligen Phase einer physiologischen Funktion mit mehreren Phasen auftreten oder mit dieser überlappen, wobei jeder der mehreren Sätze von Schlagintervallen aus der identifizierten Mehrzahl von Schlagintervallen ausgewählt wird.

7. System nach Anspruch 6, wobei der Intervallauswahlcode ferner dazu programmiert ist, Untermengen der Schlagintervalle in jeweilige Phasengruppierungen zu gruppieren, basierend darauf, in welcher Phase der mehreren Phasen der physiologischen Funktion die jeweiligen Schlagintervalle zeitlich liegen, und
wobei eine Instanz des Durchschnittsberechnungscodes, des Differenzberechnungscodes und des Variationsberechnungscodes separat aufgerufen wird, um ein jeweiliges Variationsmaß über die interessierende Oberfläche hinweg für jede der Phasengruppierungen zu bestimmen.

8. System nach einem der Ansprüche 1-7, wobei die interessierende Oberfläche eine äußere Oberfläche des Körpers eines Patienten ist und die Variationskarte eine Körperoberflächenvariationskarte über die äußere Oberfläche hinweg ist.

9. System nach Anspruch 8, ferner umfassend Rekonstruktionscode, der dazu programmiert ist, die Variationskarte auf eine interessierende Herzoberfläche zu projizieren, um eine Variationskarte der Herzoberfläche bereitzustellen.

10. System nach einem der Ansprüche 1-7, wobei die interessierende Oberfläche eine interessierende Herzoberfläche innerhalb des Körpers eines Patienten ist, die jeweiligen elektrophysiologischen Herzsignale rekonstruierte elektrophysiologische Signale an jeweiligen Orten über die interessierende Herzoberfläche hinweg basierend auf elektrophysiologischen Signalen, die nicht-invasiv über eine Oberfläche des Körpers des Patienten hinweg über ein oder mehrere Zeitintervalle gemessen werden, und Geometriedaten sind und der Variationsberechnungscode dazu programmiert ist, eine Variationskarte der Herzoberfläche über die interessierende Herzoberfläche hinweg zu erzeugen.

11. System nach einem der Ansprüche 1-7, ferner umfassend:
eine Anordnung von Körperoberflächenelektroden, die dazu ausgelegt sind, die elektrophysiologischen Herzsignale nicht-invasiv von einer äußeren Oberfläche des Körpers eines Patienten zu messen, wobei die Anweisungen ferner Rekonstruktionscode umfassen, der dazu programmiert ist, elektrophysiologische Signale an Orten zu rekonstruieren, die über die interessierende Oberfläche innerhalb des Körpers des Patienten hinweg verteilt sind, um die elektrophysiologischen Daten bereitzustellen, die elektrophysiologische Herzsignale an jeweiligen Orten über die interessierende Oberfläche hinweg repräsentieren, basierend auf den gemessenen elektrophysiologischen Herzsignalen und Geometriedaten.

12. System nach einem der Ansprüche 1-11, wobei die Anweisungen Datenanalysecode beinhalten, der dazu programmiert ist:
mindestens eine Zielstelle basierend auf der Variationskarte zu identifizieren;
eine Anleitung zum Anwenden einer Behandlung am Ziel bereitzustellen.

13. System nach Anspruch 12, wobei:
die Behandlung eine erste Behandlung ist und, nachdem die erste Behandlung angewendet wurde, aktualisierte elektrophysiologische Herzsignale über die interessierende Oberfläche hinweg bereitgestellt werden, der Intervallauswahlcode, der Durchschnittsberechnungscode, der Differenzberechnungscode und der Variationsberechnungscode jeweils dazu programmiert sind, ein aktualisiertes Variationsmaß über die interessierende Oberfläche hinweg basierend auf den aktualisierten elektrischen physiologischen Signalen zu bestimmen, und
der Datenanalysecode ferner dazu programmiert ist, eine Anleitung zum Anwenden einer zweiten Behandlung an demselben Zielort oder einem anderen Zielort basierend auf dem aktualisierten Variationsmaß oder basierend auf einer Differenz zwischen dem aktualisierten Variationsmaß und dem Variationsmaß vor dem Anwenden der ersten Behandlung bereitzustellen.

14. System nach einem der Ansprüche 1-13, ferner umfassend Ausgabegeneratorcode, der dazu programmiert ist, Ausgabedaten zu erzeugen, um eine grafische Darstellung der Variationskarte anzuzeigen.

## Revendications

1. Système (102) comprenant :
un ou plusieurs supports non transitoires lisibles par ordinateur pour stocker des données et des instructions lisibles par machine, les données comprenant des données électrophysiologiques représentatives de signaux électrophysiologiques cardiaques à des emplacements respectifs sur une surface d'intérêt ; et
une unité de traitement (114) pour accéder aux supports non transitoires lisibles par ordinateur et exécuter les instructions lisibles par machine, les instructions lisibles par machine comportant :
un code de sélecteur d'intervalle (116) programmé pour sélectionner plusieurs ensembles d'intervalles de battement, chaque ensemble d'intervalles de battement comportant des signaux électrophysiologiques cardiaques respectifs à des emplacements respectifs sur la surface d'intérêt pour l'intervalle de battement respectif ;
un code de calculateur de moyenne (118) programmé pour déterminer un intervalle de battement moyen pour chaque ensemble d'intervalles de battement sur la base des signaux électrophysiologiques cardiaques respectifs dans l'ensemble respectif d'intervalles de battement ;
un code de calculateur de différence (120) programmé pour déterminer, pour chaque ensemble d'intervalles de battement, une différence entre chacun des signaux électrophysiologiques cardiaques respectifs et l'intervalle de battement moyen pour chaque ensemble respectif d'intervalles de battement ; et
un code de calculateur de variation (122) programmé pour calculer une mesure de variation pour chacun des emplacements respectifs sur la surface d'intérêt sur la base de l'intervalle de battement moyen et des différences déterminées pour les intervalles de battement respectifs sur la surface d'intérêt, dans lequel une carte de variation sur la surface d'intérêt est générée sur la base de la mesure de variation calculée pour chacun des emplacements respectifs.

2. Système selon la revendication 1, dans lequel :
le code de calcul de différence est programmé pour calculer un écart type sur les intervalles de battement sur la base des signaux électrophysiologiques cardiaques respectifs et de la moyenne déterminée pour chaque ensemble d'intervalles de battement, et
le code de calcul de variation est programmé pour calculer la mesure de variation pour chacun des emplacements respectifs sur la base d'un quotient de l'écart type et d'une amplitude de l'intervalle de battement moyen calculé pour chacun des emplacements respectifs.

3. Système selon l'une quelconque des revendications 1 ou 2, dans lequel le code de sélecteur d'intervalle est en outre programmé pour :
faire correspondre les signaux électrophysiologiques cardiaques pour chaque emplacement respectif à une fenêtre de modèle d'intervalle pour fournir chacun des ensembles d'intervalles de battement ; et
grouper les signaux électrophysiologiques cardiaques respectifs pour chaque ensemble des intervalles de battement sur la base d'une valeur de correspondance déterminée pour chaque ensemble respectif d'intervalles de battement.

4. Système selon l'une quelconque des revendications 1 ou 2, dans lequel le code de sélecteur d'intervalle est en outre programmé pour aligner chacun des signaux électrophysiologiques cardiaques respectifs dans chaque ensemble d'intervalles de battement sur la base d'une caractéristique de morphologie de signal.

5. Système selon la revendication 4, dans lequel les signaux électrophysiologiques cardiaques respectifs sont représentatifs de l'un parmi une tachycardie ventriculaire, un rythme sinusal normal ou une tachycardie auriculaire, et la caractéristique de morphologie du signal est un point ou une région situé entre des pics R adjacents des signaux électrophysiologiques cardiaques respectifs.

6. Système selon l'une quelconque des revendications 1 ou 2, dans lequel le code de sélecteur d'intervalle est en outre programmé pour :
identifier une pluralité d'intervalles de battement qui se produisent au niveau, ou en chevauchement, d'une phase respective d'une fonction physiologique ayant de multiples phases, dans lesquels chacun des multiples ensembles d'intervalles de battement est sélectionné à partir de la pluralité d'intervalles de battement identifiés.

7. Système selon la revendication 6, dans lequel le code de sélecteur d'intervalle est en outre programmé pour regrouper des sous-ensembles des intervalles de battement en groupements de phases respectifs sur la base de celle des multiples phases de la fonction physiologique dans laquelle les intervalles de battement respectifs résident dans le temps, et
dans lequel une instance du code de calcul de moyenne, du code de calcul de différence et du code de calcul de variation est appelée séparément pour déterminer une mesure de variation respective sur la surface d'intérêt pour chacun des groupements de phases.

8. Système selon l'une quelconque des revendications 1 à 7, dans lequel la surface d'intérêt est une surface extérieure du corps d'un patient, et la carte de variation est une carte de variation de surface corporelle sur la surface extérieure.

9. Système selon la revendication 8, comprenant en outre un code de reconstruction programmé pour projeter la carte de variation sur une surface cardiaque d'intérêt pour fournir une carte de variation de surface cardiaque.

10. Système selon l'une quelconque des revendications 1 à 7, dans lequel la surface d'intérêt est une surface cardiaque d'intérêt à l'intérieur du corps d'un patient, les signaux électrophysiologiques cardiaques respectifs sont des signaux électrophysiologiques reconstruits à des emplacements respectifs sur la surface cardiaque d'intérêt sur la base de signaux électrophysiologiques mesurés de manière non invasive sur une surface du corps du patient sur un ou plusieurs intervalles de temps et de données géométriques, et le code de calculateur de variation est programmé pour générer une carte de variation de surface cardiaque sur la surface cardiaque d'intérêt.

11. Système selon l'une quelconque des revendications 1 à 7, comprenant en outre :
un agencement d'électrodes de surface corporelle conçues pour mesurer les signaux électrophysiologiques cardiaques de manière non invasive à partir d'une surface externe du corps d'un patient, les instructions comprenant en outre un code de reconstruction programmé pour reconstruire des signaux électrophysiologiques sur des emplacements répartis sur la surface d'intérêt dans le corps du patient afin de fournir les données électrophysiologiques représentatives des signaux électrophysiologiques cardiaques à des emplacements respectifs sur la surface d'intérêt sur la base des signaux électrophysiologiques cardiaques mesurés et de données géométriques.

12. Système selon l'une quelconque des revendications 1 à 11, dans lequel les instructions comportent un code d'analyse de données programmé pour :
identifier au moins un site cible sur la base de la carte de variation ;
fournir des conseils pour l'application d'un traitement à la cible.

13. Système selon la revendication 12, dans lequel :
le traitement est un premier traitement, et après que le premier traitement a été appliqué, des signaux électrophysiologiques cardiaques mis à jour sont fournis sur la surface d'intérêt,
chacun du code de sélecteur d'intervalle, du code de calculateur de moyenne, du code de calculateur de différence et du code de calculateur de variation étant programmés pour déterminer une mesure de variation mise à jour sur la surface d'intérêt sur la base des signaux électrophysiologiques mis à jour, et
le code d'analyse de données est également programmé pour fournir des conseils pour appliquer un second traitement au même emplacement cible ou à un emplacement cible différent sur la base de la mesure de variation mise à jour ou sur la base d'une différence entre la mesure de variation mise à jour et la mesure de variation avant d'appliquer le premier traitement.

14. Système selon l'une quelconque des revendications 1 à 13, comprenant en outre un code générateur de sortie programmé pour générer des données de sortie pour afficher une représentation graphique de la carte de variation.
